# EUROPEAN PATENT APPLICATION

(11) **EP 4 840 430 A1**
(43) Date of publication of application: **18.11.2026**
(21) Application number: 25176169.8
(22) Date of filing: 13.05.2025
(51) Int. Cl.: A61F 13/15, A61F 13/539, A61F 13/514

(54) **ABSORBENT ARTICLE WITH BACKSHEET-TO-CORE ADHESIVE PATTERN**

(71) Applicant: The Procter & Gamble Company, Cincinnati, OH 45202 (US)
(72) Inventor: LUNA VALVERDE, Lucia Paloma, 65824 Schwalbach am Taunus (DE); RINNERT, Thorsten, 65824 Schwalbach am Taunus (DE)
(74) Representative: P&G Patent Germany

(57) **Abstract**

An absorbent article (20) comprising a topsheet (24), an absorbent core (28) and a backsheet (25), and a backsheet-to-core adhesive pattern comprising a front adhesive zone (100) disposed adjacent the front edge (10) of the article and forming a front end seal between the topsheet and the backsheet; a first longitudinally-extending adhesive zone (104) and a second longitudinally-extending adhesive zone (106) disposed underneath the absorbent core and respectively adjacent the first and second longitudinal edges of the core (284, 286); a back adhesive zone (102) adjacent the back edge (12) of the article and forming a back end seal between the topsheet and the backsheet; and a first non-adhesive zone (110) disposed in the back gap (22) between the back edge (282) of the absorbent core and the back edge (12) of the article. The bonding pattern reduces adhesive migration event on the wearer-facing side of the diaper. The article may further comprise one of an apertured topsheet at least in a central portion (240) of the article, a back waist cuff (28) and a front waistband (36).

## Description

### FIELD OF THE INVENTION

The invention relates to personal hygiene absorbent articles of the type worn in the crotch region of an individual, such as baby diapers, training pants, feminine sanitary pads and/or adult incontinence articles. The present invention relates more particularly to the backsheet-to-core adhesive pattern on the inward-facing side of the backsheet.

### BACKGROUND OF THE INVENTION

Absorbent articles for personal hygiene of the type indicated above are designed to absorb and contain body exudates, in particular small or large quantity of urine. Absorbent articles comprise typically several layers providing different functions, in particular a wearer-facing liquid permeable topsheet, a garment-facing liquid impermeable backsheet comprising a polymeric film, and an absorbent core in-between, among other components. The absorbent core can absorb and retain exudates for a prolonged amount of time, minimizing re-wet to keep the wearer dry and avoid soiling of clothes or bed sheets.

Various absorbent cores have been suggested in the art. For a long time, marketed absorbent articles typically comprised a blend of comminuted wood pulp with superabsorbent polymer (SAP) particles as absorbent material, see for example US 5,151,092 (Buell). Absorbent cores having an absorbent material consisting essentially of SAP ("airfelt-free" cores) have been more recently proposed, see for example WO95/11652 (Tanzer), US6,790,798 (Suzuki), WO2008/155699 (Hundorf), or WO2012/052172 (Van Malderen). Absorbent cores with channels have also been proposed, the channels increasing the fluid acquisition properties of the core and/or acting as a folding guide. WO2012/170778 (Rosati et al.) discloses absorbent structures that comprise superabsorbent polymers, optionally a cellulosic material, and at least a pair of substantially longitudinally-extending channels (see also WO2012/170779, WO2012/170781 and WO2012/170808).

Modern absorbent articles also comprise a pair of longitudinal cuffs along the longitudinal edges of the article. The longitudinal cuffs comprise in the crotch portion of the article an elasticized free edge that can extend vertically outwardly of the topsheet to form a pair of longitudinal seals at the inguinal regions of the wearer. Absorbent articles comprising a back waist cuff adjacent the back edge of the article have been proposed, see e.g. WO2020/242714 (Schneider et al.), WO 2020/242715 (Raycheck et al.). The back waist cuff has an opening on its inward-facing edge so that the back waist cuff forms a pocket for catching fecal matter. The back waist cuff may be preferably elasticized but some articles have a non-elasticized waist cuff. Absorbent articles may also comprise an elasticized front waistband disposed adjacent to the front edge of the article, see e.g. WO2021/225832A1 (Kuilbacak et al.). The back waist cuff and the front waistband can be elasticized by comprising an elastic film or elastic threads sandwiched between two nonwovens that form the cuff and/or waistband.

The various components of an absorbent article are attached to another so that they stay in place before and during usage. Typical attachment means are mechanical bonding, heat bonding, ultrasonic bonding and adhesive bonding. The attachment means are chosen by the manufacturer to balance costs of the equipments, cost of the material and performance required. The absorbent core is typically attached to the backsheet by gluing.

The inward-facing side of the backsheet film is typically applied with an adhesive in a series of longitudinally regularly spaced adhesive tracks. The hotmelt adhesive may typically be sprayed on the backsheet by a plurality of adhesive nozzles disposed in a row before attaching the absorbent core to form a regular backsheet-to-core adhesive pattern. Alternative backsheet-to-core gluing patterns have been proposed. WO2012/170341A1 (Hippe) discloses a diaper having a reduced backsheet-to-core adhesive pattern. In Hippe, the absorbent core is attached to the backsheet only in certain, limited areas. More recently, WO2016/183304A1 (Bianchi et al.) discloses an improved backsheet-to-core adhesive pattern having a pre-determined backsheet-to-core peel strength range.

The backsheet-to-core adhesive pattern typically extends beyond the absorbent core to at least partially form the front seal and back seal between the topsheet and the backsheet, preventing delamination of the absorbent article at the front and back edges of the article (end flaps). The backsheet-to-core adhesive pattern of the prior art thus typically extends longitudinally along the full length of the absorbent article, including the back gap area extending from the back edge of the absorbent core to the back edge of the article.

The inventors have now found that the backsheet-to-core adhesive may sometimes seep through the back gap area onto the topsheet. This is undesirable for several reasons. As the article is typically bi-folded, the adhesive can cause the topsheet to bond to itself so that the article cannot be unfolded without being damaged. The adhesive can also cause the topsheet to accidentally bond to the back waist cuff so that the cuff can no longer perform its waste capturing function. This adhesive seepage may be especially problematic for apertured topsheet and/or topsheet having a relatively low basis weight. In the context of apertured or low basis weight topsheet, this will also lead to waist cuff sticking and the waist pocket will become unfunctional as it stays closed.

There is therefore a need for backsheet-to-core adhesive pattern to avoid bleed through of the adhesive onto the topsheet while still efficiently anchoring the core as well as sealing the chassis endflaps.

### SUMMARY OF THE INVENTION

The present invention is directed to absorbent articles) extending in a longitudinal direction (x), a transversal direction (y) and a vertical direction (z), the absorbent article comprising a liquid permeable topsheet on a wearer-facing side, a liquid impermeable backsheet film on a garment-facing side. The absorbent core is disposed between the topsheet and the backsheet, wherein the absorbent core comprises a front edge, a back edge and a first and second longitudinal edges. The topsheet and the backsheet delimit a front edge and a back edge of the article. The back edge of the absorbent core is spaced from the back edge of the absorbent article by a back gap. The article comprises an adhesive disposed on the core-facing side of the backsheet ("backsheet-to-core adhesive") according to a backsheet-to-core adhesive pattern. The backsheet-to-core adhesive pattern comprises:
- a front adhesive zone disposed adjacent the front edge of the article and forming a front end seal between the topsheet and the backsheet;
- a first longitudinally-extending adhesive zone and a second longitudinally-extending adhesive zone disposed respectively adjacent the first and second longitudinal edges of the core, and underneath the absorbent core;
- a back adhesive zone adjacent to the back edge of the article and forming a back end seal between the topsheet and the backsheet; and
- a first non-adhesive zone disposed in the back gap between the back edge of the absorbent core and the back edge of the article.

The backsheet-to-core adhesive pattern is particularly useful for an absorbent article comprising a topsheet that is at least apertured in a portion of the topsheet that is vertically superposed with first non-adhesive zone. The backsheet-to-core adhesive pattern is also particularly useful when the article comprises a transversally-oriented elastic back waist cuff disposed above the topsheet and adjacent the back edge of the article, the back waist cuff comprising a pocket portion that can extend away from the topsheet to form a pocket for receiving waste matter and the pocket portion of the back waist cuff at least partially vertically overlaps with the first non-adhesive zone disposed in the back gap between the back edge of the absorbent core and the back edge of the article.

The present invention is also directed to a process for making an absorbent article according to the invention. The backsheet-to-core adhesive may be applied by non-contact applicators, typically a plurality of adhesive spray nozzles providing one of large swirls, mini swirls or random adhesive patterns in machine direction. The backsheet-to-core adhesive may be in particular applied by a plurality of spray nozzles disposed in a row in cross-machine direction, wherein at least some of the nozzles are turned on and off to provide the predetermined backsheet-to-core pattern comprising longitudinally extending adhesive portion of different lengths (intermittent applications of the adhesive).

The absorbent article may optionally comprise a urine indicator applied on the backsheet separately from the backsheet-to-core adhesive. The urine indicator may also be applied as a hotmelt adhesive, typically by a contact applicator. The urine indicator composition usually comprises a pH indicator that changes color when contacted with urine.

### BRIEF DESCRIPTION OF THE DRAWINGS

Fig. 1 shows the core-facing side of a backsheet of an absorbent article (diaper) with a backsheet-to-core adhesive pattern according to the invention, as well as a urine indicator; the absorbent core and front waistband and back waist cuff are shown in dashed lines;
Fig. 2 shows a simplified top view of the wearer-facing side of the article of Fig. 1, showing a front elastic waistband and a back waist cuff;
Fig. 3 shows a schematic exploded view of some of the main components of an absorbent article according to the invention, including an acquisition distribution system that was not shown in Figs. 1-2;
Fig. 4 shows a schematic cross-section of the absorbent article as in Figs. 1-3 towards the front edge of the article;
Fig. 5 shows a schematic cross-section of an absorbent article as in Figs. 1-2 in the crotch region of the article;
Fig. 6 shows a schematic cross-section of an absorbent article as in Figs. 1-2 through the free-standing portion of the back waist cuff;
Fig. 7 shows a schematic cross-section of an absorbent article as in Figs. 1-2 through the adhesively bonded region of the waist cuff adjacent the back edge of the article;
Fig. 8 shows the core-facing side of a backsheet with an alternative backsheet-to-core adhesive pattern;
Fig. 9 is a schematic sketch of a process for applying the backsheet-to-core adhesive on a continuous backsheet material;
Figs 10 and 11 schematically illustrate different applicators that may be used to apply the backsheet-to-core adhesive to the backsheet.

### DETAILED DESCRIPTION OF THE INVENTION

### Introduction

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 21 °C +/- 2 °C and 50 +/- 20% Relative Humidity (RH).

The invention will now be further illustrated with reference to the embodiments as described in the Figures. For ease of discussion, the absorbent article and its components will be discussed with reference to the numerals referred to in these Figures. However, it should be understood that these exemplary embodiments and the numerals are not intended to limit the scope of the claims, unless specifically indicated.

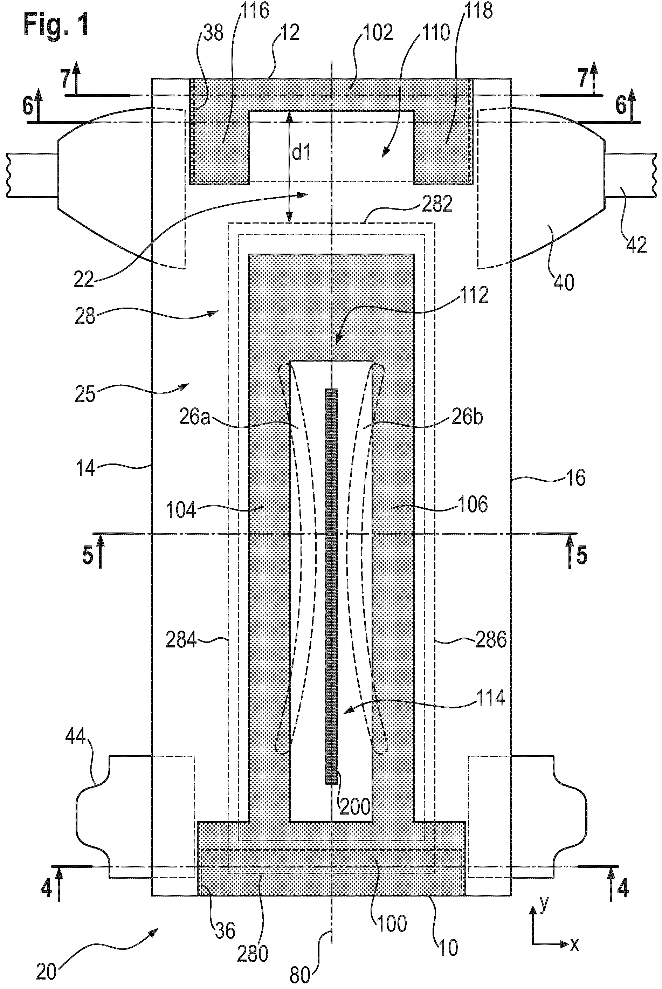

### General description of Fig. 1

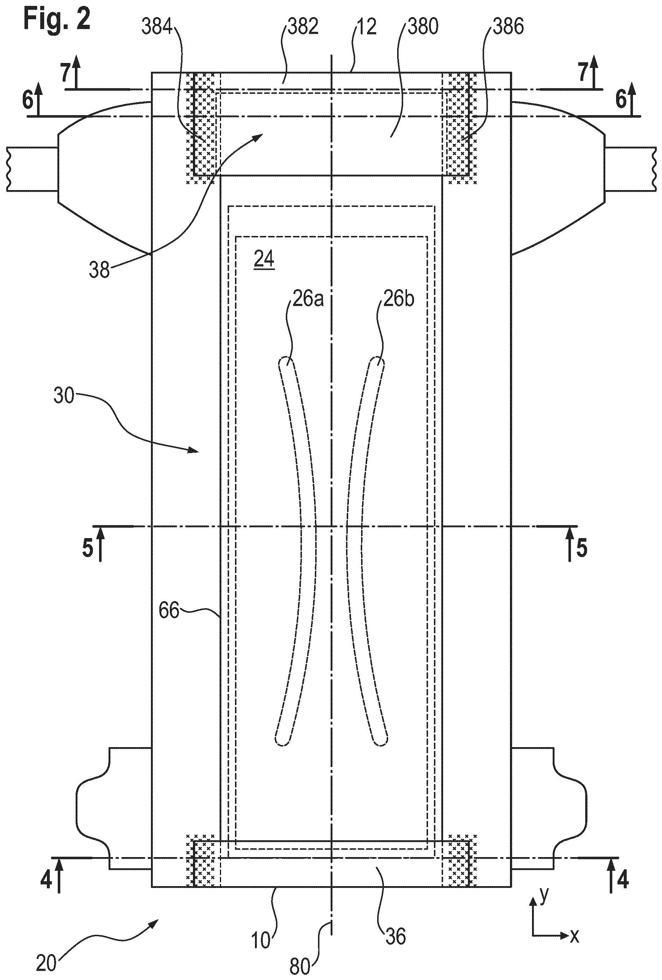

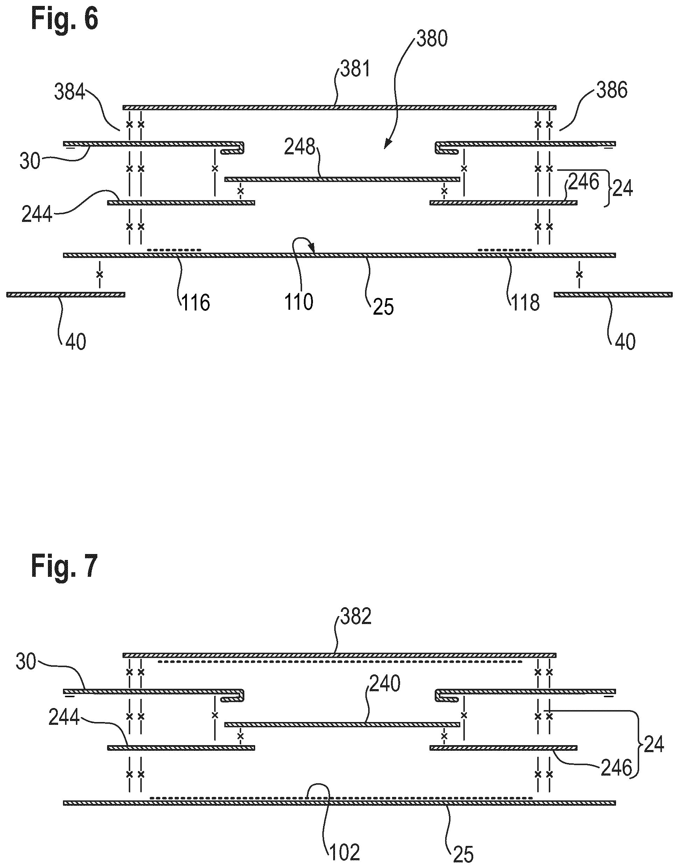

Fig. 1 shows an exemplary backsheet-to-core adhesive pattern according to the invention. For readability, the backsheet 25 is represented in continuous line and the contour of the absorbent core 28 and of the core channels 26a, 26b in broken lines, with other layers of the articles such as the topsheet not displayed. The article represented is a so-called taped diaper, which comprise back ears 40 with tapes 42 which can be releasably affixed to a landing zone (not shown) on the front waist of the garment-facing side of the article. The absorbent article represented also comprises front ears 44 which provide a better coverage of the diaper along the front waist of the user. Of course, the backsheet-to-core adhesive pattern of the invention may also be used in pant diapers, which have pre-sealed side edges or taped diapers have unibody chassis without separate back and front ears components. Additional layers and components of the article are shown in Figs. 2-7, as will be discussed further below.

The absorbent article 20 comprises a front edge 10, a back edge 12, and two longitudinally extending side (lateral) edges 14, 16. The front edge 10 is the edge of the article which is intended to be placed towards the front of the user when worn, and the back edge 12 is the opposite edge. The absorbent article is notionally (i.e. virtually) divided by a longitudinal axis 80 extending from the front edge to the back edge of the article and dividing the article in two substantially symmetrical halves relative to this axis, when viewing the core in the plane formed by the longitudinal direction (y) and the transversal direction (x). The longitudinal direction extends along the length of the article, and the transversal direction is perpendicular to the longitudinal direction.

The exemplarily absorbent article is represented in a flat state in Figs. 1-2 in a plane parallel to transversal direction (x) and the longitudinal direction (y). If some part of the article is under tension due to elasticized components, the article may be typically flattened using clamps along the periphery of the article and/or a sticky surface, so that the topsheet and backsheet can be pulled taut. Closed articles such as training pants may be cut open along the side seams so that they can be stretched flat on a surface. Unless otherwise indicated, dimensions and areas disclosed herein apply to the article in this flat-out configuration. The article has a length L measured along the longitudinal axis 80 from the middle of the back edge to the middle of the front edge.

The backsheet 25 may be generally rectangular as shown in Fig. 1. Shaped backsheet having a narrower waist thus forming an hour-glass shape are also known and may be used in the invention. Such shaped backsheet may inherently form the front and back ears thus eliminating the need for additional material for these components. However this construction has other disadvantages such as having to cut-out materials resulting in waste and making it more difficult to elasticize the back ears for example.

The absorbent core 28 comprises in the example shown a pair of channels 26a, 26b shown in broken lines on Fig. 1. The channels 26a, 26b are generally longitudinally extending and may be mirror image of each other relative to the longitudinal axis. The channels may be curved, as shown in Fig. 1, but this is not limiting the present invention. The absorbent core may comprise straight channels, in particular channels that are straight and orientated in the longitudinal direction, or the absorbent core may be free of any such channels. The backsheet-to-core adhesive pattern will now be described.

### Backsheet-to-core adhesive 100, 102, 104, 106, 116, 118

The backsheet-to-core adhesive pattern comprises a front adhesive zone 100 adjacent the front edge 10 of the article and a back adhesive zone 102 adjacent the back edge of the article. The front adhesive zone and the back adhesive zone are typically transversally oriented, meaning they are longer in the transversal direction (x) than the in the longitudinal direction (y). The front and back adhesive zones in particular at least partially contribute to forming a seal between the topsheet and backsheet of the article along the front and back edges of the article respectively, also referred to as chassis endflaps in the art. The front and back end seals may also comprise mechanical or heat seals formed by compressing the topsheet and backsheet along a plurality of point bonds (not represented), the point bonds being disposed in a plurality of lines oriented in the longitudinal direction, or otherwise, as is known in the art.

The back adhesive zone 102 that at least partially forms the backend seal between the backsheet and the topsheet may further comprise longitudinal extensions 116, 118 adjacent and inwards of the longitudinal edges of the back waist cuff 38. These adhesive extension zones may further collaborate with other bonding means such as the fusion bonds 384, 386 of the back waist cuff to improve the attachment of the back waist cuff to the rest of the article.

The backsheet-to-core adhesive comprises a first non-adhesive zone 110 disposed in the back gap 22 between the absorbent core's back edge 282 and the article back edge 12. The first non-adhesive zone 110 reduces the chance that the backsheet-to-core adhesive seeps up to the wearer-surface of the absorbent article. When the article comprises a transversally-oriented elastic back waist cuff 38 disposed above the topsheet and adjacent the back edge 12 of the article, the first non-adhesive zone 110 preferably overlaps with the pocket portion 380 of the cuff that is not bonded to the topsheet and that can extend away from the topsheet to form a pocket for receiving waste matter. The length d1 of the non-adhesive zone 110 as measured on the longitudinal centerline 80 may typically be in the range of from 1 cm to 20 cm, in particular in the range of from 2 cm to 12 cm.

The backsheet-to-core adhesive further comprises a core adhesive zone disposed between the absorbent core and the backsheet. The core adhesive zone comprises at least a first longitudinally-extending adhesive zone 104 and a second longitudinally-extending adhesive zone 106 respectively disposed on each side of the longitudinal axis 80 and adjacent the first and second longitudinal edges of the core 284, 286.

The core adhesive zone may optionally comprise a second non-adhesive zone 114 disposed between the first and second longitudinally-extending adhesive zones 284, 286 of the core adhesive zone. The second non-adhesive zone 114 is disposed underneath the absorbent core and advantageously at least partially overlaps with the core channels 26a, 26b when these are present to reduce stress on the backsheet when the absorbent core swells and these channels form three-dimensional channels. The second non-absorbent zone 114 may optionally comprise a urine indicator, for example a stripe 200 of urine indicator composition along the longitudinal axis 80 of the absorbent article. These urine indicator compositions are typically applied on the backsheet or the absorbent core as a hotmelt adhesive comprising a pH indicator. It may also be beneficial to apply the hotmelt urine indicator in a zone of the backsheet or the core that does not overlap with the backsheet-to-core adhesive to avoid any negative interactions between the two adhesives.

The backsheet-to-core adhesive pattern may comprise a generally rectangular shape consisting of the front adhesive zone 100, the two longitudinally-extending adhesive zones 104, 106 and a back core adhesive zone 112 disposed between the longitudinally-extending zones 104 106 adjacent the back edge 282 of the core, as illustrated in Fig 1. Advantageously, the back core adhesive zone 112 is disposed between the core 28 and the backsheet 25 between the two longitudinally-extending adhesive zones 104, 106 and does not extend beyond the back edge 282 and the side edges 284, 286 of the core wrap. In this way, the absorbent core covers completely the core adhesive back zone so that the backsheet-to-core adhesive is prevented from seeping through the topsheet in this area. In other words, the absorbent core can shield the topsheet from the backsheet-to-core adhesive disposed underneath and prevent the adhesive from reaching the surface of the topsheet.

The dimensions of the adhesive zones may generally vary and depend on the type of article considered, as well as the dimensions of the absorbent core, its channels and back waist cuff and front waistband in present. The backsheet-to-core adhesive is typically intermittently applied to provide a series of longitudinally aligned stripes of different lengths and intermittently interrupted. It is understood that the longitudinal stripes forming the backsheet-to-core adhesive zones are typically not continuous stripe of adhesive but are rather applied as spirals or other non-continuous applicator mean to be economical in adhesive material.

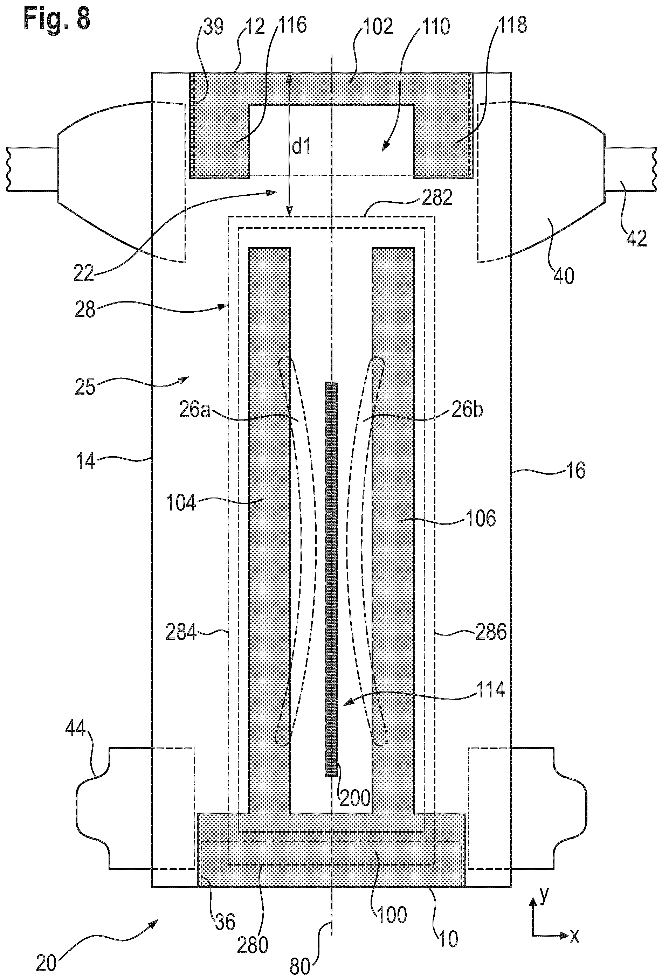

### Alternative design (Fig. 8)

While the second non-adhesive zone 114 underneath the absorbent core may be entirely surrounded by a rectangularly shaped portion of the backsheet-to-core adhesive formed by the front adhesive zone 100, the two longitudinally-extending adhesive zones 104, 106 and the back core adhesive zone 112, other backsheet-to-core designs are possible. An alternative backsheet-to-core adhesive pattern is illustrated in Fig. 8 and differs from the pattern of Fig. 1 in that the back core adhesive zone 112 was omitted. In this alternative design, there is no backsheet-to-core adhesive between the first non-adhesive zone 110 and the second non-adhesive zone 114. This alternative pattern may reduce the amount of backsheet-to-core adhesive used while still sufficiently anchoring the absorbent core to the backsheet.

### Process for applying the first adhesive and the second adhesive

The backsheet-to-core adhesive may be applied as known in the art in an industrial manufacturing process by using a series of adhesive nozzles disposed in cross-direction. The nozzles can be rapidly switched on and off to apply the backsheet-to-core adhesive on the backsheet in the desired pattern before assembling the backsheet with other components of the diaper. The backsheet-to-core adhesive nozzles are typically non-contact applicators. Suitable nozzles are for example discussed in WO2016/183304 for a different adhesive pattern but which principle can be re-applied for the present invention. Other processes and variations thereof may of course be used.

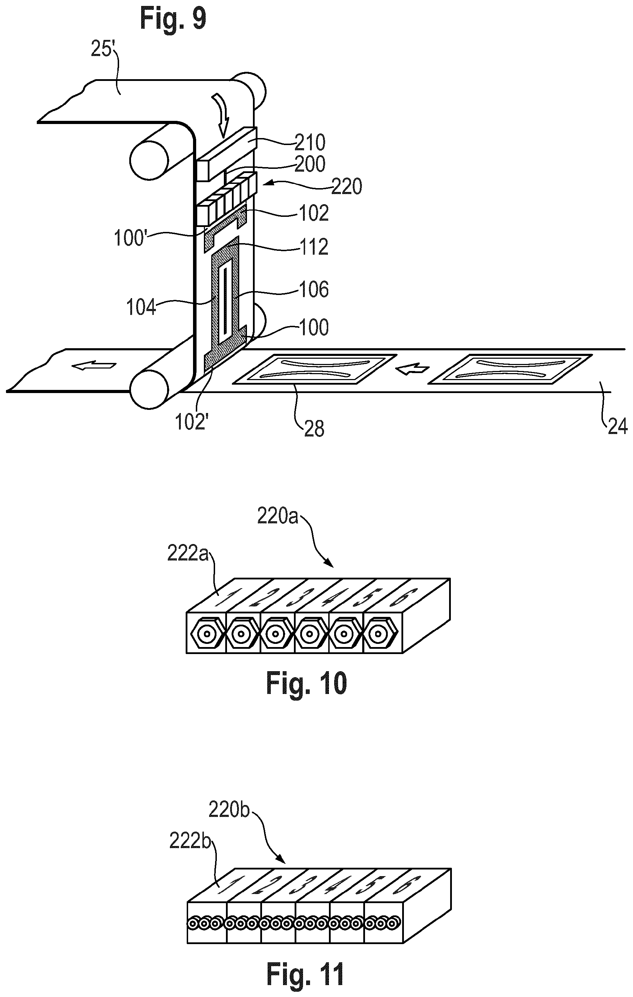

The backsheet-to-core adhesive and the urine indicator, if present, may be applied as schematically represented on Fig. 9, although other processes and variations thereof may of course be used. The process for making and assembling the rest of the absorbent article may be according to any known processes in the art and will not be further detailed herein. The exemplary process of Fig. 9 shows the backsheet coming as a first continuous web feed 25' from the left side of the Figure and passing successively through two glue applicators. The urine indicator applicator 210 applies the urine indicator composition, typically a hotmelt with a pH indicator, to form the urine indicator 200 on the backsheet. The urine indicator applicator is advantageously a contact glue applicator, in particular for simplicity and cost a slot-coater 210 through which the slot glue is applied directly onto the backsheet. In slot coating, the adhesive exits the applicator through a thin, wide passageway - the nozzle laying the adhesive directly down on top of the substrate. The glue stripe can vary in width and pattern, depending on the application needs.

Directly after the urine indicator, a backsheet-to-core applicator 220 applies the backsheet-to-core adhesive according to the backsheet-to-core application pattern. The backsheet-to-core adhesive applicator may be in particular a non-contact applicator that comprises a plurality of nozzles 222a,b installed in parallel in cross-direction, as represented on Figs. 10 and 11. In the example described, the backsheet-to-core adhesive applicator comprises six nozzles which can be independently controlled and turned on and off to form the desired backsheet-to-core adhesive pattern, for example as represented in Figs. 1,8.

The individual spray nozzles of the backsheet-to-core adhesive applicator may be of any type known in the art. In a first applicator example 220a, as illustrated on Fig. 10, the nozzles each deliver a single large swirl (also called spiral) from each nozzle 222a. These nozzles are e.g. available from Nordson under the designation "CF applicator". The width of such large spirals may for example range from 10 mm to 30 mm.

In such example of an applicator having six nozzles 222a installed in parallel in cross-machine direction, the desired adhesive backsheet-to-core pattern can be obtained by intermittently turning on and off selected nozzles as the backsheet web passes through the applicator 220. For example, for the pattern exemplified in Fig. 1, the nozzles 1-6 are all turned on to form the front adhesive zone 100, then all the nozzles except the two nozzles 2 and 5 are switched off, with the adhesive dispensed from nozzles 2 and 5 forming the longitudinal-extending adhesive zones 104, 106, then nozzles 3 and 4 are switched on to close the rectangular core adhesive zone by applying the adhesive in the back core adhesive zone 112. The nozzles are then all switched off to form the beginning of back gap 22, then nozzles 1 and 6 are switched on to form the adhesive extensions 116 and 118, and all 6 nozzles are again switched on to form the back adhesive zone 102.

Other applicators than the large spiral applicator 222a may be used according to the same principle of having a plurality of nozzles disposed in parallel in cross-machine direction. The backsheet-to-core adhesive may be in particular applied by an applicator 220b comprising several nozzle units 222b each having a plurality of so-called mini-swirl nozzles, in particular three or four sub-nozzles per unit. These mini-swirl nozzles are for example available from Nordson under the "Summit" designation. Each unit 222b may apply a plurality of swirls having together the same width as indicated previously for one large swirl/spiral pattern 222a. These nozzle units 222b may also be independently turned on and off to provide the desired area of coverage and glue pattern as described above. Mini-swirls can be used at a faster speed than large swirl/spiral and have a better edge definition of the boundary of the glue pattern, while not being as precise as slot application.

Other known suitable spraying nozzle types may be used, for example nozzle spraying a random pattern of glue, such as those supplied by Nordson under the "Signature" spray nozzle designation. These nozzles produce random patterns of glue in fibrous form with relatively low peel forces for each individual glue fiber but high shear forces due to the plurality of bonding points. Although randomly sprayed, the adhesive filaments may appear as being generally longitudinally aligned due to the movement of the web at high speed, helping better distribute the stress along the boundary of the glue pattern and prevent spot bonding of the film between the gaps of the glue filament. An air flow may be used in conjunction with a spray nozzle to direct or disperse the glue filaments. The backsheet-to-core adhesive may also be applied by a plurality of contact applicators, in particular slot glue applicators, as is known in the art.

The backsheet-to-core adhesive pattern is of course preferably applied on the continuous backsheet material web 25' so that the front adhesive zone 100 of one article is formed continuously with the back adhesive zone 102' of the next article and vice versa.

It may be advantageous, as represented, to apply the urine indicator 200 before the backsheet-to-core adhesive. Otherwise, there is a risk of smearing the backsheet-to-core adhesive on the contact applicator of the urine indicator.

After the adhesives have been applied, the feed of absorbent cores, as shown coming from the right, is synchronized with the backsheet-to-core adhesive patterns on the backsheet material web 25'. The backsheet and absorbent core are faced and pressure is applied so that the core is adhesively attached to the backsheet. The feed of absorbent cores is typically supported on a continuous substrate which may be another component of the article, such as the remaining components of the article which have been pre-assembled on the line, with the topsheet 24 being the largest of these components on which other components such as the cuff and acquisition distribution layer or layers 52, 54 have been assembled. The absorbent articles are individualized downstream by die cutting in a separate step (not shown) as is known in the art.

Further attachment means between components of the articles are non-exhaustively represented in the cross-sectional drawings as broken horizontal lines for adhesives and "x" signs for fusions bonds, for example the front ears 44 may be bonded to the backsheet 25 by adhesive chassis side slots 46. The longitudinal glue slots used for the elastic barrier cuff threads 340 adjacent the longitudinal edges 13, 14 can also form the longitudinal seals between the backsheet and the topsheet and/or barrier leg cuffs in the absorbent articles. These glues and other fusion or mechanical bonded are partially represented in the Figures and may be as for any conventional absorbent articles.

### Adhesive composition

The backsheet-to-core adhesive, as well as the other adhesives used in the article, may be any type of adhesives known in the art. Hotmelt adhesives are generally used for absorbent articles and can be used to form the desired backsheet-to-core application pattern. Hotmelt adhesives generally include one or more polymers to provide cohesive strength (e.g., aliphatic polyolefins such as ethylene-propylene copolymers, polyetheramides, polyetheresters, ethylene vinyl acetate copolymers, styrene-butadiene or styrene-isoprene block copolymers, and combinations thereof etc...), a resin or analogous material (called a tackifier) to provide adhesive strength (e.g. hydrocarbons distilled from petroleum distillates, rosins and/or rosin esters, terpenes derived for example from wood or citrus, etc.), and optional waxes, plasticizers and other materials to modify viscosity (e.g. mineral oil, polybutene, paraffin oils, ester oils, and the like), and/or other additives including, but not limited to, antioxidants or other stabilizers. Exemplary suitable commercial adhesives for the backsheet-to-core adhesives and other adhesives that may be used in the invention are available from Fuller under reference number 1286 or 1358, or from Henkel under reference number DM 526, DM538 or DM3800.

The backsheet-to-core adhesive may be typically applied at a basis weight of between 1 g/m² to 20 g/m², in particular from 1 g/m² to 10 g/m² taking as reference the weight of the adhesive divided by the full backsheet film surface. The total amount of backsheet-to-core adhesive in an article (in particular a diaper) may for example range from 10 mg to 200 mg per article, in particular from 20 mg to 120 mg depending on the size of the article (for example about 60 mg for a size 4 baby diaper).

### General description of an absorbent article 20

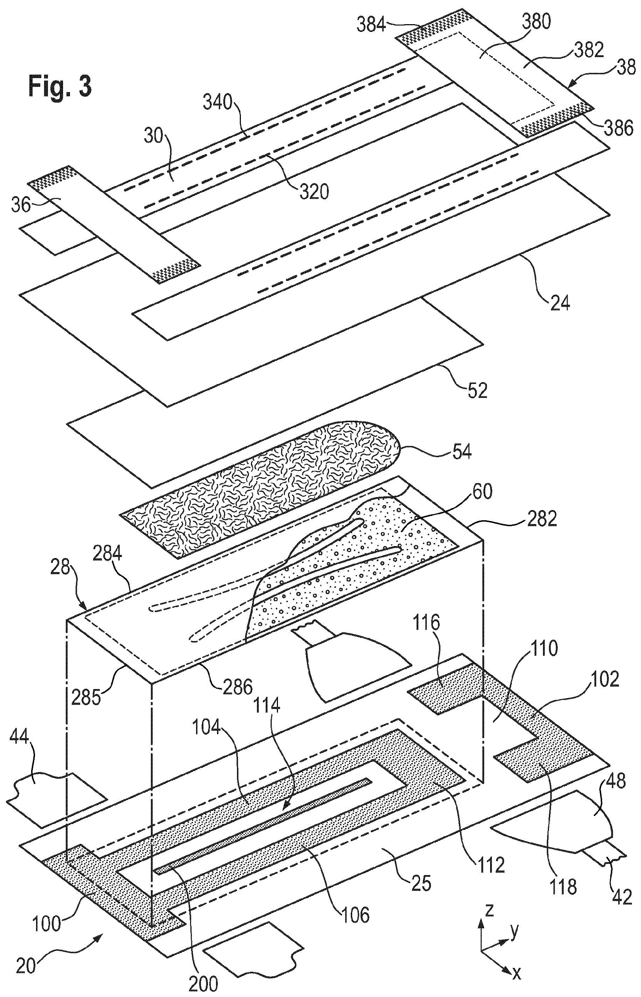

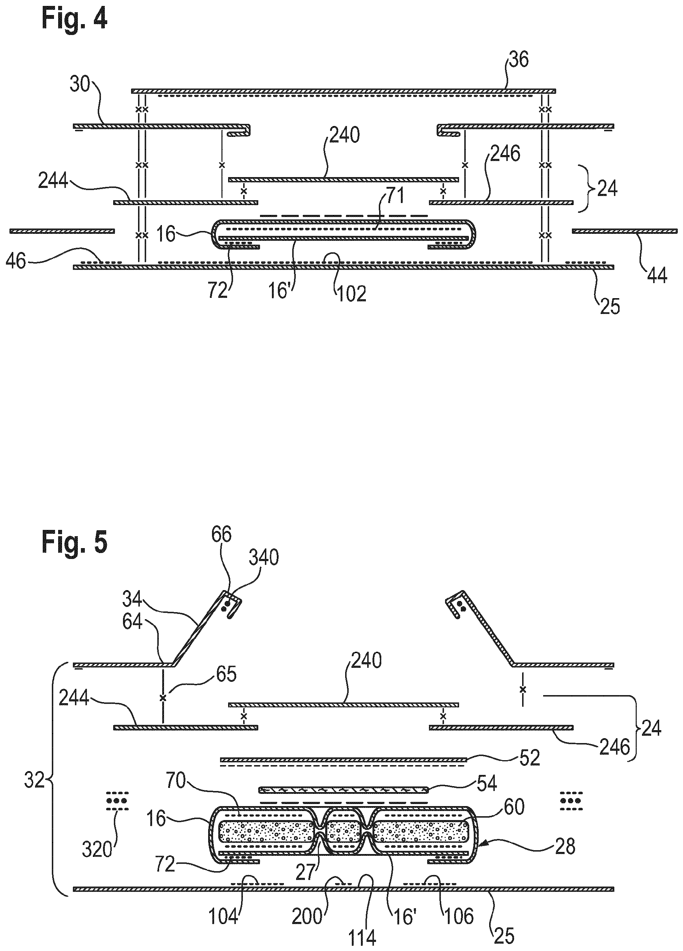

The absorbent article 20 comprises a liquid permeable topsheet 24, a liquid impermeable backsheet 25 and an absorbent core 28 between the topsheet 24 and the backsheet 25. Some typical components of a baby taped diaper 20 are further represented in the exploded view of Fig. 3, and in cross-section view in Figs. 4-7.

Typically all components will be attached to the other neighboring components by adhesive, heat and pressure bonding, or otherwise, but only the backsheet-to-core adhesive pattern is represented in these Figures for readability.

The absorbent article may also comprise further typical components such as an acquisition layer 52 and/or a distribution layer 54, longitudinally-aligned elasticized gasketing cuffs 34 on the side of the chassis, and partially upstanding barrier leg cuffs 32. The Figures also show other typical taped diaper components such as a fastening system comprising fastening tabs 42 attached towards the back edge 12 of the article and cooperating with a landing zone towards the front edge 10 of the article. The absorbent article may also comprise other typical components, which are not represented in the Figures, such as transverse barrier cuffs, a topsheet lotion, various graphics including a patterned urine indicator, etc...

The topsheet 24, the backsheet 25, the absorbent core 28 and the other article components may be assembled in a variety of well-known configurations, in particular by gluing and/or heat embossing. Exemplary diaper assemblies are for example generally described in US3,860,003, US5,221,274, US5,554,145, US5,569,234, US5,580,411, and US6,004,306. The absorbent article is preferably thin. The article may be advantageously thin at the intersection of the longitudinal and transversal axes (i.e. the center of the article), for example with a caliper of from 1.0 mm to 8.0 mm, in particular from 1.5 mm to 6.0 mm, as measured using the Caliper Test described below.

Some components of the article will now be discussed in more detail.

### Topsheet 24

The topsheet 24 is preferably compliant, soft-feeling, and non-irritating to the wearer's skin. Further, at least a portion of the topsheet 24 is liquid permeable, permitting liquids to readily penetrate through its thickness. Typical topsheet comprise or consist of a nonwoven material.

Conventional nonwoven topsheet having a basis weight in the range of from about 10 g/m² to about 28 g/m² may be used in the invention. The invention is particularly useful when the topsheet is a low basis weight nonwoven such as spunbond having a basis weight in the range from about 12 g/m² to about 18 g/m² as these materials may be prone to adhesive seep-through that the present invention is preventing.

Alternatively, the present invention is also particularly useful when the topsheet is apertured. The topsheet 24 may comprise a plurality of apertures to ease penetration of exudates there-through, such as urine and/or feces (solid, semi-solid, or liquid). Examples of apertured topsheet are disclosed in US 6,632,504. The present invention was found to be particularly beneficial when at least a portion of the topsheet comprises apertures, as the claimed backsheet-to-core adhesive pattern of the invention can reduce the seepage of the adhesive through the topsheet by avoiding that any substantial portion of the apertured topsheet be vertically superposed with the backsheet-to-core adhesive.

Other topsheet materials may also be used, in a particular multi-component topsheet comprising a central apertured portion and two nonapertured lateral portions. The topsheet may be a multi-component topsheet as represented in Figs. 4-7 comprising a central portion 240 which is apertured and two side portions 244, 246 which are not apertured. Such multi-component topsheet construction is economical as the central apertured potion is typically a high basis weight nonwoven (basis weight in the range of from 25 g/m² to 50 g/m²) which is more expensive than the non-apertured nonwoven material of the side portions (from 10 g/m² to 20 g/m²). The side portions may be substantially covered by the barrier cuff material 30 in the assembled article, and thus the lower basis weight material may not significantly be visible in use. The side portions may thus generally have about the same length or be shorter in the transversal direction than the barrier cuff material 30. An exemplary multi-component topsheet that may be used in the invention is for example disclosed in WO2018/170729A1.

The multi-component topsheet may comprise:
i) a first discrete substrate 244 having an inner lateral edge;
ii) a second (central) discrete substrate 240; and
iii) a third discrete substrate 246 having an inner lateral edge;
wherein the second discrete substrate is disposed at least partially intermediate the first discrete substrate and the third discrete substrate in the transversal direction. The inner lateral edges of the first and second discrete substrates at least partially overlap the second discrete substrate. The second discrete substrate is joined to the first discrete substrate in a first bonding zone, and the second discrete substrate is joined to the third discrete substrate in a second bonding zone. The first, second, and third discrete substrates may be of equal length in the longitudinal direction. The second discrete substrate may be a relatively high basis weight (in the range of 20 g/m² to 50 g/m²), apertured, nonwoven material. The first and third discrete substrate may be more affordable non-apertured nonwovens having a lower basis weight in the range of 10 g/m² to 20 g/m².

The skilled person understands that apertures are different from the inter-fibrous pores that are found in conventional nonwovens. Rather apertures are engineered openings in the material of the topsheet having predetermined dimensions and placement. Apertures may have any shapes, typically oval or circular, having an average surface as measured on the surface of the topsheet of at least 0.5 mm² typically up to 10 mm² for most application wherein viscous exudates are desired to be absorbed in the article.

The topsheet 24 forms the majority of the wearer-facing side of the absorbent article and is directly in contact with the wearer's skin during use. The topsheet 24 can be joined to the backsheet 25, the absorbent core 28 and/or any other layers as is known in the art (as used herein, the term "joined" encompasses configurations whereby an element is directly secured to another element by affixing the element directly to the other element, and configurations whereby an element is indirectly secured to another element by affixing the element to intermediate member(s) which in turn are affixed to the other element). Usually, the topsheet 24 and the backsheet 25 are joined directly to each other in some locations (e.g. on, or close to, the periphery of the article) and are indirectly joined together in other locations through one or more other elements of the article 20.

Other suitable topsheet include formed film topsheet, which are described in US3,929,135, US 4,324,246, US 4,342,314, US 4,463,045, and US 5,006,394. Other suitable topsheet may be made in accordance with US 4,609,518 and US 4,629,643. Such formed films are available from The Procter & Gamble Company of Cincinnati, Ohio as "DRI-WEAVE" and from Tredegar Corporation, based in Richmond, VA, as "CLIFF-T".

Any portion of the topsheet may be coated with a lotion as is known in the art. Examples of suitable lotions include those described in US5,607,760, US5,609,587, US5,643,588, US5,968,025 and US6,716,441. The topsheet 24 may also include or be treated with antibacterial agents, some examples of which are disclosed in WO95/24173. Further, the topsheet, the backsheet or any portion of the topsheet or backsheet may be embossed and/or matte finished to provide a more cloth like appearance.

### Backsheet 25

The backsheet 25 is the component of the absorbent article 20 which forms the majority of the garment-facing surface of the article when worn by the user. The backsheet is typically directly in contact with the bottom side 16' of the core and prevents the exudates absorbed and contained therein from soiling bed sheets and undergarments. The backsheet 25 comprises a polymeric film that is impermeable to liquids (e.g. urine). The backsheet 25 typically comprises a thin plastic film on the exterior surface of which a thin nonwoven is adhesively attached to improve the tactile feel of the backsheet (backsheet laminate). Exemplary backsheet films include those manufactured by Tredegar Corporation, based in Richmond, VA, and sold under the trade name CPC2 film. Suitable backsheet materials may include breathable materials which permit vapors to escape from the article, while still preventing exudates from passing through the backsheet 25. Exemplary breathable materials include microporous films such as manufactured by Mitsui Toatsu Co., of Japan under the designation ESPOIR NO and by Tredegar Corporation of Richmond, VA, and sold under the designation EXAIRE, and monolithic films such as manufactured by Clopay Corporation, Cincinnati, OH under the name HYTREL blend P18-3097. Some breathable composite materials are described in greater detail in WO 95/16746 (E. I. DuPont), US 5,938,648 (LaVon et al.), US 4,681,793 (Linman et al.), US 5,865,823 (Curro), US 5,571,096 (Dobrin et al.) and US 6,946,585 (London Brown).

### General description of the absorbent core 28

As used herein, the term "absorbent core" refers to a component of the absorbent article which comprises an absorbent material enclosed in a core wrap. As used herein, the term "absorbent core" does not include the topsheet, the backsheet and (if present) an acquisition-distribution layer or multilayer system, which is not integral part of the absorbent core, in particular which is not placed within the core wrap. The absorbent core is typically the component of an absorbent article that has the most absorbent capacity of all the components of the absorbent article and which comprises all, or at least the majority of, superabsorbent polymer (SAP). The core may consist essentially of, or consist of, the core wrap, the absorbent material and adhesives. The terms "absorbent core" and "core" are herein used interchangeably.

The absorbent core may typically be generally rectangular with a width Wc in the transversal direction and a length Lc in the longitudinal direction as measured from edge to edge, including the regions of the core wrap which do not comprise absorbent material, such as the front and back ends 280, 282 of the core wrap which may be adhesively sealed to another. If the core is not rectangular, the maximum core wrap dimensions are measured along the transversal and longitudinal direction and can be used to report the length and width of the core. The width and length of the core may vary depending on the intended usage. For baby and infant diapers, the width Wc may for example in the range from 40 mm to 200 mm and the length Lc from 100 mm to 500 mm. The longitudinal axis of the core may be contiguous with the longitudinal axis 80 of the article.

The absorbent core comprises a front edge 280, a back edge 282 and two longitudinally extending side edges 284, 286 joining the front edge and the back edge. The front edge of the core is the edge of the core intended to be placed towards the front edge of the absorbent article. Typically the absorbent material 60 of the core may be advantageously distributed in somewhat higher amount towards the front edge than towards the back edge as more absorbency is typically required towards the front half of the article. Typically the front and back edges 280, 282 of the core may be shorter than the side edges 284, 286 of the core. The absorbent core may also comprise a top side 16 and a bottom side 16'. The top side of the core is the side placed or intended to be placed towards the topsheet 24 of the article and the bottom side is the side placed or intended to be placed towards the backsheet 25 in the finished article. The top side of the core wrap is typically more hydrophilic than the bottom side.

The following will provide an exemplary description of possible core components. Further details are described for example in WO2012/170778 (Rosati et al.), WO2014/93311A1 (Arizti et al), WO2014/093310 (Ehrnsperger et al.), which disclose absorbent structures that comprise superabsorbent polymers, optionally a cellulosic material, and at least a pair of substantially longitudinally extending channels.

### Core wrap 16, 16'

The core wrap may comprise a first substrate 16 generally forming the top side of the core and a second substrate 16' generally forming the bottom side of the core wrap (the same reference numbers are used for the material and the side of the core wrap). The first and second substrates may be two different materials, as shown in the cross-sections Figures 4-7. but any other known core wrap constructions may also be used, for example wherein the core wrap is formed of a single material with one single longitudinal seal. The first and second substrates can be attached by gluing or otherwise to form at least one C-wrap seal 72 along each of the side edges 284, 286 of the core. The first and second substrates may be a nonwoven web, such as a laminate comprising spunbond ("S") or meltblown ("M") layer. For example spunmelt polypropylene nonwovens are suitable, in particular those having a laminate web SMS, or SMMS, or SSMMS, structure, and having a basis weight range of about 5 g/m² to 15 g/m². Suitable materials are for example disclosed in US 7,744,576, US 2011/0268932 A1, US 2011/0319848 A1 and US 2011/0250413 A1. The bottom substrate 16' may be inherently hydrophobic but air-permeable, and the top substrate 16 may be hydrophillically treated. There may be an end seal 71 along the front edge 282 and back edge 280 of the core wrap.

Combining the auxiliary adhesive layer with a C-wrap seal along at least one and preferably two longitudinal edges of the core, and optionally a further fibrous adhesive web (not represented), can provide an immobilization of the absorbent material in dry and wet state. The absorbent core may in general advantageously achieve an SAP loss of no more than about 70%, 60%, 50%, 40%, 30%, 20%, or 10% according to the Wet Immobilization Test described in US2010/0051166A1.

### Absorbent material 60

The absorbent material in the core can be any absorbent material known in the art. Such absorbent material typically comprise a superabsorbent polymer.

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test as indicated in EDANA method NWSP 241.0.R2 (19). The SAP may in particular have a CRC value of at least 20 g/g, or more than 24 g/g, or of from 20 g/g to 50 g/g, or from 20 g/g to 40 g/g, or 24 g/g to 35 g/g. "Superabsorbent polymer particles", as used herein, refers to a superabsorbent polymer material which is in particulate form so as to be flowable in the dry state.

The fluid permeability of a superabsorbent polymer can be quantified using its Urine Permeability Measurement (UPM) value, as measured in the test disclosed European patent application EP3,391,963A1. The UPM of the SAP may for example be of at least 10 x10⁻⁷ cm3.sec/g, or at least 15, 20, 30, 40, or 50 x10⁻⁷ cm3.sec/g.

The absorbent material may comprise or consist of a mixture of SAP and cellulose fibers, typically comprising from 60% to 80% by weight of SAP by weight of the absorbent material. Alternatively, the absorbent material may also comprise a relatively high proportion of superabsorbent polymer (herein abbreviated as "SAP") within the core wrap. The SAP content may represent in particular at least 85%, 90%, 95% and up to 100% of the weight of the absorbent material. The absorbent material may in particular comprise no or only small amount of cellulose fibers, such as less than 20%, in particular less than 10%, 5% or even 0% of cellulose fibers by weight of the absorbent material. The absorbent material may thus consist or consist essentially of SAP. The SAP may be typically in particulate forms (superabsorbent polymer particles), but it is not excluded that other forms of SAP may be used such as a superabsorbent polymer foam or superabsorbent fibers for example. The absorbent core may thus be relatively thin, in particular thinner than conventional cores comprising cellulosic fibers. In particular, the caliper of the core (before use) as measured at the geometric center of the core according to the Caliper Test as described herein may be from 0.25 mm to 5.0 mm, in particular from 0.5 mm to 4.0 mm.

The absorbent material layer may have a generally rectangular shape when seen from the top but other shapes are possible as is known in the art, such as a "T", "Y", "hourglass" or "dog-bone" shape. In particular, the deposition area may show a tapering along its width at the crotch region of the core. In this way, the absorbent material deposition area may have a relatively narrow width in an area of the core intended to be placed in the crotch region of the absorbent article. This may provide for example better wearing comfort.

The absorbent core may comprise one or more channel, referred herein as a couple of channels 26a,b. The term "channel" designates a longitudinally extending area of the core comprising less absorbent material than the surrounding areas so that an insulting fluid can be quickly distributed along the channel towards the front and back of the core. The channels may be in particular substantially free of absorbent material. By "substantially free" it is meant that in each of these areas the basis weight of the absorbent material is at least less than 25%, in particular less than 20%, less than 10%, of the average basis weight of the absorbent material in the rest of the absorbent material deposition area of the core. In particular there can be no absorbent material in these areas 26ab. Minimal amount such as involuntary contaminations with absorbent material particles that may occur during the making process are not considered as absorbent material. The channels 26 are advantageously surrounded by the absorbent material, when considering the plane of the core, which means that the channel areas 26 do not extend to any of the edges of the deposition area 8 of the absorbent material 60.

The top layer 16 and the bottom layer 16' of the core wrap may be bonded to each other through channels 26a,b. The bond 27 between the substrates in these area may be at least partially formed by an auxiliary adhesive 70 applied directly to the inner surface of at least one of the substrate, but other bonding methods are not excluded. This bonding allows channels 26 to form more pronounced three-dimensional channels 26' as the absorbent material swells when it absorbs a liquid such as urine. Examples of channels according to the invention are described in details for example in WO2012/170778 (Rosati et al.), WO2014/93311A1 (Arizti et al), WO2014/093310 (Ehrnsperger et al.) which disclose absorbent structures that comprise superabsorbent polymers, optionally a cellulosic material, and at least a pair of substantially longitudinally extending channels.

When the absorbent material 60 swells upon absorbing a liquid, the core wrap bonds 27 remain at least initially attached in the channel areas 26. The absorbent material 60 swells in the rest of the core when it absorbs a liquid, so that the core wrap forms one or more pronounced three-dimensional channels along the core wrap bond 27. These three-dimensional channels can distribute an insulting fluid along their length to a wider area of the core. The channels provide a quicker fluid acquisition speed and a better utilization of the absorbent capacity of the core.

The absorbent core 28 illustrated comprise a first and second channels 26ab disposed on each side of the longitudinal axis 80. It is not excluded that the core may also comprise more than two channels or alternatively a single channel. Shorter channel areas substantially free of absorbent material may also be present, for example in the back region or the front region of the core, as seen for example in the Figures of WO2012/170778.

The channels extend substantially longitudinally, which means typically that each area extends at least as much in the longitudinal direction (y) than in the transversal direction (x), and typically at least twice as much in the longitudinal direction than in the transverse direction (as measured after projection on the respective axis). The channels 26 may have a length L' projected on the longitudinal axis 80 of the core that is at least 10% of the length Lc of the absorbent core, in particular from 20% to 80%. The channels may have an area substantially free of absorbent material having a width along at least part of their length which is at least 2 mm, or at least 3 mm or at least 4 mm, up to for example 20 mm, or 16 mm or 12 mm. The width may be constant through substantially the whole length or may vary along the length of the channels.

The channels 26 may be curved as shown in the Figures but they may be also straight and parallel to the longitudinal axis. It may be advantageous that there is no channels that coincide with the longitudinal axis 80' of the core. When present as a pair of channels 26a,b, these may be spaced apart from one another over their whole longitudinal dimension. The smallest spacing distance may be for example at least 5 mm, or at least 10 mm, or at least 16 mm.

The absorbent material 60 may be deposited on any of the substrates 16, 16' using known techniques In particular the SAP printing technology as disclosed for example in US2006/024433 (Blessing), US2008/0312617 and US2010/0051166A1 (both to Hundorf et al.), which allow relatively precise deposition of SAP at relatively high speed may be used. This technique uses a transfer device such as a printing roll to deposit SAP onto a substrate disposed on a grid of a support which may include a plurality of cross bars extending substantially parallel to and spaced from one another. Channel areas 26 substantially free of absorbent material can be formed for example by modifying the pattern of the grid and receiving drums so that no SAP is applied in the selected areas, as exemplary disclosed in US2012/0312491 (Jackels). This technology allows highspeed and precise deposition of SAP on a substrate in particular to provide one or more area(s) 26 substantially free of absorbent material surrounded by absorbent material. US2014/027066 (Jackels) further discloses specific raised strips and mating strips on the equipment for bonding the core substrates through the channel areas.

The absorbent material may be substantially continuously distributed in the deposition area. By "substantially continuous" it is meant that at least 50%, or at least to 70% and up to 100% of the deposition area comprises a continuous layer of absorbent material as seen from the top side of the core. The absorbent material may be for example applied as a single continuous layer on one of the substrate, the layer thus directly forming the material deposition area. A continuous layer of absorbent material, in particular of SAP, may also be obtained by combining two absorbent layers having matching discontinuous absorbent material application pattern wherein the resulting layer is substantially continuously distributed across the absorbent material deposition area, as exemplarily taught in US2008/0312622A1 (Hundorf).

As disclosed in this reference, the absorbent core 28 may also comprise a fibrous thermoplastic adhesive material, to further immobilize the absorbent material 60 during the making process of the core and usage of the article. The fibrous thermoplastic adhesive material may be in particular useful to immobilize a dual layers of absorbent material to their respective substrate 16, 16'. Each of these absorbent layers may comprise land areas separated by junction areas and the fibrous thermoplastic adhesive material may then be at least partially in contact with the absorbent material in the land areas and at least partially in contact with the substrate layer 16, 16' in the junction areas. This imparts an essentially three-dimensional net-like structure to the fibrous layer of thermoplastic adhesive material, which in itself is essentially a two-dimensional structure of relatively small thickness, as compared to the dimension in length and width directions. Thereby, the fibrous thermoplastic adhesive material may provide cavities to cover the absorbent material in the land areas, and thereby immobilizes this absorbent material. The fibrous adhesive may be for example sprayed on an absorbent layer after it has been deposited on its substrate during the core making process.

The article further comprises a liquid permeable topsheet 24 and a liquid impermeable backsheet 25 with the absorbent core 28 positioned between the topsheet and the backsheet.

### Acquisition and distribution layers 52, 54

The absorbent articles of the invention may comprise an acquisition layer 52, a distribution layer 54, both, or a single layer having both functions of acquiring and distributing the fluid. Typically, these layers do not comprise SAP as this may slow the acquisition and distribution of the fluid. The prior art discloses many type of acquisition-distribution system, see for example WO 2000/59430 (Daley), WO 95/10996 (Richards), US 5,700,254 (McDowall), WO 02/067809 (Graef).

The function of an acquisition layer is to quickly acquire the fluid away from the topsheet so as to provide a good dryness for the wearer. The acquisition layer is typically placed directly under the topsheet. If present, the distribution layer may be at least partially disposed under the acquisition layer. The acquisition layer may typically be or comprise a non-woven material, for example a SMS or SMMS material, comprising a spunbonded, a melt-blown and a further spunbonded layer or alternatively a carded chemical-bonded nonwoven. The non-woven material may in particular be latex bonded. Exemplary upper acquisition layers 52 are disclosed in US 7,786,341. Carded, resin-bonded nonwovens may be used, in particular where the fibers used are solid round or round and hollow PET staple fibers (50/50 or 40/60 mix of 6 denier and 9 denier fibers). An exemplary binder is a butadiene/styrene latex. Nonwovens have the advantage that they can be manufactured outside the converting line and stored and used as a roll of material. Further useful nonwovens are described in US 6,645,569 (Cramer et al.), US 6,863,933 (Cramer et al.), US 7,112,621 (Rohrbaugh et al.), US 2003/148684 (Cramer et al.) and US 2005/008839 (Cramer et al.).

The acquisition layer may be stabilized by a latex binder, for example a styrene-butadiene latex binder (SB latex). Processes for obtaining such latices are known, for example, from EP 149880 (Kwok) and US 2003/0105190 (Diehl et al.). In certain embodiments, the binder may be present in the acquisition layer 52 in excess of about 12%, about 14% or about 16% by weight. SB latex is available under the trade name GENFLO^{™} 3160 (OMNOVA Solutions Inc.; Akron, Ohio).

A distribution layer 54 may also be present. The function of a distribution layer is to spread the insulting fluid liquid over a larger surface within the article so that the absorbent capacity of the core can be more efficiently used. Typically the distribution layer is made of a nonwoven material based on synthetic or cellulosic fibers and having a relatively low density. The density of the distribution layer may vary depending on the compression of the article, but may typically range from 0.03 to 0.25 g/cm³, in particular from 0.05 to 0.15 g/cm³ measured at 0.30 psi (2.07kPa). The distribution layer 54 may also be a material having a water retention value of from 25 to 60, preferably from 30 to 45, measured as indicated in the procedure disclosed in US 5,137,537. The distribution layer 54 may typically have an average basis weight of from 30 to 400 g/m², in particular from 100 to 300 g/m². As shown in Fig. 2, the distribution layer may be rounded towards the back of the article. The distribution layer may be also profiled so that its basis weight towards the back of the article is lower than towards the front. The distribution layer may optionally comprise channels that are at least partially superposed with the core channels for example as disclosed in EP3,238,679A1 (Bianchi et al.).

The distribution layer may for example comprise at least 50% by weight of crosslinked cellulose fibers. The crosslinked cellulosic fibers may be crimped, twisted, or curled, or a combination thereof including crimped, twisted, and curled. This type of material has been used in the past in disposable diapers as part of an acquisition system, for example US 2008/0312622 A1 (Hundorf). The crosslinked cellulosic fibers provide higher resilience and therefore higher resistance against the compression in the product packaging or in use conditions, e.g. under baby weight.

### Fastening system

The absorbent article may include a fastening system. The fastening system can be used to provide lateral tensions about the circumference of the absorbent article to hold the absorbent article on the wearer. This fastening system is not necessary for pant article since the waist region of these articles is already bonded and elasticized. The fastening system usually comprises a fastener 42 such as tape tabs, hook and loop fastening components, interlocking fasteners such as tabs & slots, buckles, buttons, snaps, and/or hermaphroditic fastening components, although any other known fastening means are generally acceptable. A landing zone is normally provided on the front waist region of the article for the tab fastener 42 to be releasably attached. Some exemplary surface fastening systems are disclosed in US 3,848,594, US 4,662,875, US 4,846,815, US 4,894,060, US 4,946,527, US 5,151,092 and US 5,221,274 (Buell). An exemplary interlocking fastening system is disclosed in US 6,432,098. The fastening system may also provide a means for holding the article in a disposal configuration as disclosed in US 4,963,140 (Robertson et al.)

The fastening system may also include primary and secondary fastening systems, as disclosed in US 4,699,622 to reduce shifting of overlapped portions or to improve fit as disclosed in US 5,242,436, US 5,499,978, US 5,507,736, and US 5,591,152.

### Front and back ears 44, 40

The absorbent article may comprise front ears 44 and back ears 40 as is known in the art. The ears can be integral part of the chassis, for example formed from the topsheet and/or backsheet as side panel. Alternatively, as represented in Fig. 3, they may be separate elements attached by gluing and / or heat embossing. The back ears 40 are advantageously stretchable to facilitate the attachment of the tabs 42 on a landing zone (not shown) on the external side of the backsheet, and maintain the taped diapers in place around the wearer's waist. The front ears 44 may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

### Barrier leg cuffs 34 and gasketing cuffs 32

Absorbent articles such as taped diapers or pant diapers typically further comprise components that improve the fit of the article around the legs of the wearer, in particular barrier leg cuffs 34 and gasketing cuffs 32. These cuffs may be formed by a piece of material 30, typically a nonwoven, which is partially bonded to the rest of the article and can be partially raised away and thus form a barrier leg cuff 34 that can stand up from the plane defined by the topsheet, as shown for example in Fig. 5. The barrier leg cuffs 34 improve containment of liquids and other body exudates approximately at the junction of the torso and legs of the wearer. The barrier leg cuffs 34 extend at least partially between the front edge and the back edge of the absorbent article on opposite sides of the longitudinal axis.

The barrier leg cuffs 34 may be delimited by a proximal edge 64 joined to the rest of the article, typically the topsheet and/or the backsheet, and a free terminal edge 66 intended to contact and form a seal with the wearer's skin. The barrier leg cuffs 34 may be joined at the proximal edge 64 with the chassis of the article by a bond 65 which may be made for example by adhesive bonding, fusion bonding or combination of known bonding means. The bond 65 at the proximal edge 64 may be continuous or intermittent.

The barrier leg cuffs 34 can be integral with (i.e. formed from) the topsheet or the backsheet, or more typically be formed from a separate material 30 joined to the rest of the article. Typically the material of the barrier leg cuffs may extend through the whole length of the article but is "tack bonded" to the topsheet towards the front edge and back edge of the article so that in these sections the barrier leg cuff material remains flush with the topsheet. Each barrier leg cuff 34 may comprise one, two or more elastic strings 340 close to this free terminal edge 66 to provide a better seal.

In addition to the barrier leg cuffs 34, the article may comprise gasketing cuffs 32, which are formed in the same plane as the chassis of absorbent article and are typically placed laterally of the barrier leg cuffs 34. The gasketing cuffs 32 improve the sealing around the thighs of the wearer. Usually each gasketing leg cuff 32 will comprise one or more elastic string or elastic element 320 comprised in the chassis of the diaper for example between the cuff material 30 and backsheet 25 in the area of the leg openings.

US3,860,003 describes a disposable diaper which provides a contractible leg opening having a side flap and one or more elastic members to provide an elasticized leg cuff (a gasketing cuff). US4,808,178 (Aziz) and US4,909,803 (Aziz) describe disposable diapers having "stand-up" elasticized flaps (barrier leg cuffs) which improve the containment of the leg regions. US4,695,278 (Lawson) and US4,795,454 (Dragoo) describe disposable diapers having dual cuffs, including gasketing cuffs and barrier leg cuffs. All or a portion of the barrier leg and/or gasketing cuffs may be treated with a lotion.

### Front waistband 36 and back waist cuff 38

The absorbent article may also comprise at least one elastic front waistband 36 to improve fit and containment. The elastic front waistband is intended to elastically expand and contract to dynamically fit the wearer's waist. The absorbent article can be constructed so as to have a front elastic band and back waist cuff. The elastic waist feature may be constructed in a number of different configurations including those described in US4,515,595, US4,710,189, US5,151,092 and US5,221,274. For example, the transversally-oriented elastic front waistband may be disposed on the topsheet adjacent the front edge of 10 of the absorbent article and bonded to the topsheet by a combination of fusion bonds at the edges of the waistband and adhesive bond along its length in the transverse direction.

As indicated previously, the backsheet-to-core adhesive pattern according to the invention is especially useful when the article further comprises a with a back waist cuff 38 disposed above the topsheet 24 adjacent the back edge 12 of the article. The back waist cuff 38 is transversally-oriented. The back waist cuff comprises a pocket portion 380 that can extend away from the topsheet to form a pocket for receiving waste matter. The pocket portion 380 of the back waist cuff at least partially vertically overlaps with the first non-adhesive zone 110 disposed in the back gap 22 between the back edge 282 of the absorbent core and the back edge 12 of the article.

The back waist cuff 38 may comprise two longitudinally-extending side edges 384, 386, an outward-facing back edge 382 and an inward-facing front edge 388, where the two longitudinally-extending side edges and the outward-facing back edges are bonded directly or indirectly to the topsheet and the inward-facing back edge is unbonded and can extend away from the topsheet to form the opening of the pocket portion of the back waist cuff. Examples of such back waist cuffs are disclosed in WO2023/196768 (Williams et al.) but other back waist cuff construction are known and can applied in the articles of the present invention. The back waist cuff is preferably at least partially comprised of an elastic material.

### Relations between the layers and components

Apart from the backsheet-to-core adhesive zone pattern described in details previously, adjacent layers may be joined together using conventional bonding method such as adhesive coating via slot coating or spraying on the whole or part of the surface of the layer, or thermo-bonding, or pressure bonding or combinations thereof. Most of the bonding between components is for clarity and readability not represented in the Figure. Bonding between the layers of the article should be considered to be present unless specifically excluded. Adhesives may be typically used to improve the adhesion of the different layers, for example between the backsheet and the core wrap. The adhesives used may be any standard hotmelt adhesive as known in the art.

### Method of making

Apart from the method for applying the gluing pattern described in details previously, the absorbent article may be made otherwise by any conventional methods known in the art. The articles may be hand-made or industrially produced at high speed on a modern converting line.

### Test procedures

The values indicated herein are measured according to the methods indicated herein below, unless specified otherwise. All measurements are performed at 21°C ± 2°C and 50% ± 20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

### Centrifuge Retention Capacity (CRC)

The CRC measures the liquid absorbed by the superabsorbent polymer particles for free swelling in excess liquid. The CRC is measured according to EDANA method WSP 241.2-05.

### Caliper Test

This test may be used to measure the caliper of a layer or an assembly such as the absorbent core or absorbent article (before use i.e. without fluid loading) in a standardized manner.

Equipment: Mitutoyo manual caliper gauge with a resolution of 0.01 mm, or equivalent instrument.

Contact Foot: Flat circular foot with a diameter of 17.0 mm (± 0.2 mm). A circular weight may be applied to the foot (e.g., a weight with a slot to facilitate application around the instrument shaft) to achieve the target weight. The total weight of foot and added weight (including shaft) is selected to provide the desired pressure, unless indicated otherwise 2.07 kPa (0.30 psi), to the sample.

The caliper gauge is mounted with the lower surface of the contact foot in an horizontal plane so that the lower surface of the contact foot contacts the center of the flat horizontal upper surface of a base plate approximately 20 x 25 cm. The gauge is set to read zero with the contact foot resting on the base plate.

Ruler: Calibrated metal ruler graduated in mm.

Stopwatch: Accuracy 1 second.

Sample preparation: The core is conditioned at least 24 hours as indicated above.

Measurement procedure: The core is laid flat with the bottom side, i.e. the side intended to be placed towards the backsheet in the finished article facing down. The point of measurement (e.g. the crotch point C) is carefully drawn on the top side of the core taking care not to compress or deform the core.

The contact foot of the caliper gauge is raised and the core is placed flat on the base plate of the caliper gauge with the top side of the core up so that when lowered, the center of the foot is on the marked measuring point.

The foot is gently lowered onto the article and released (ensure calibration to "0" prior to the start of the measurement). The caliper value is read to the nearest 0.01 mm, 10 seconds after the foot is released.

The procedure is repeated for each measuring point. If there is a fold at the measuring point, the measurement is done in the closest area to this point but without any folds. Ten samples are measured in this manner for a given product and the average caliper is calculated and reported with an accuracy of one tenth mm.

### Misc.

Dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article (20) extending in a transversal direction (x), a longitudinal direction (y) and a vertical direction (z), the absorbent article comprising:
- a liquid permeable topsheet (24);
- a liquid impermeable backsheet (25) comprising a backsheet film;
- an absorbent core (28) between the topsheet and the backsheet, wherein the absorbent core comprises a front edge (280), a back edge (282) and a first and second longitudinal edges (284, 286);
wherein the topsheet and the backsheet delimit a front edge (10) and a back edge (12) of the article;
wherein the back edge (282) of the absorbent core is spaced from the back edge (12) of the absorbent article by a back gap (22);
- a backsheet-to-core adhesive disposed on the absorbent core-facing side of the backsheet film according to a backsheet-to-core adhesive pattern, the backsheet-to-core adhesive pattern comprising:
a front adhesive zone (100) disposed adjacent the front edge (10) of the article and forming a front end seal between the topsheet and the backsheet;
a first longitudinally-extending adhesive zone (104) and a second longitudinally-extending adhesive zone (106) disposed underneath the absorbent core and respectively adjacent the first and second longitudinal edges of the core (284, 286);
a back adhesive zone (102) adjacent the back edge (12) of the article and forming a back end seal between the topsheet and the backsheet; and
a first non-adhesive zone (110) disposed in the back gap (22) between the back edge (282) of the absorbent core and the back edge (12) of the article.

2. The absorbent article (20) according to claim 1, wherein the backsheet-to-core adhesive pattern comprises a second non-adhesive zone (114) between the first and second longitudinally-extending adhesive zones (104, 106) underneath the absorbent core (28).

3. The absorbent article (20) according to claim 1 or 2, wherein the backsheet-to-core adhesive pattern comprises a back core adhesive zone (112) disposed between the longitudinally-extending zones (104,106) adjacent the back edge (282) of the core, preferably wherein said back core adhesive zone (112) does not extend beyond the back edge (282) of the core wrap.

4. The absorbent article (20) according to claim 1 or 2, wherein there is no backsheet-to-core adhesive between the first non-adhesive zone (110) and the second non-adhesive zone (114)

5. The absorbent article (20) according to any of the preceding claims, wherein at least a portion (240) of the topsheet (24) that overlaps with the absorbent core (28) is apertured and/or wherein the topsheet is a low basis weight nonwoven having a basis weight in the range of from 10 g/m² to 20 g/m².

6. The absorbent article according to the preceding claim, wherein the topsheet is a multi-component topsheet comprising:
- i) a first discrete substrate (244) having an inner lateral edge;
- ii) a second discrete substrate (240); and
- iii) a third discrete substrate (246) having an inner lateral edge;
wherein the second discrete substrate is disposed at least partially intermediate the first discrete substrate and the third discrete substrate in a longitudinal direction, so that the inner lateral edges of the first and second discrete substrates at least partially overlap the second discrete substrate, wherein the second discrete substrate is joined to the first discrete substrate in a first bonding zone, and wherein the second discrete substrate is joined to the third discrete substrate in a second bonding zone, wherein the second discrete substrate is an apertured nonwoven material having a basis weight comprised in the range of 20 g/m² to 50 g/m².

7. The absorbent article according to any of the preceding claims, wherein the absorbent core (28) comprises an absorbent material (60) within a core wrap (16, 16'), the core wrap comprising a top side (16) oriented towards the topsheet and a bottom side (16') oriented towards the backsheet.

8. The absorbent article according to claim 7, wherein the absorbent core comprises at least one longitudinally-extending channel (26a,b) surrounded by absorbent material (60) in the plane formed by the longitudinal and transversal directions, wherein the channel is substantially free of absorbent material, preferably wherein the top side of the core wrap is bonded to the bottom side of the core wrap through the channel.

9. The absorbent article according to any of the preceding claims, wherein the article further comprises:
- a transversally-oriented back waist cuff (38) disposed above the topsheet and adjacent the back edge (12) of the article, the back waist cuff comprising a pocket portion (380) that can extend away from the topsheet to form a pocket for receiving waste matter, preferably wherein the pocket portion (380) of the back waist cuff at least partially vertically overlaps with the first non-adhesive zone (110) disposed in the back gap (22) between the back edge (282) of the absorbent core and the back edge (12) of the article.

10. The absorbent article according to the preceding claim, wherein the back waist cuff (38) comprises two longitudinally-extending side edges (384, 386), an outward-facing back edge (382) and an inward-facing front edge (388), where the two longitudinally-extending side edges and the outward-facing back edges are bonded directly or indirectly to the topsheet and the inward -facing back edge is unbonded and can extend away from the topsheet to form the opening of the pocket portion of the back waist cuff.

11. The absorbent article according to any of the preceding claims, further comprising a transversally-oriented elastic front waistband (36) disposed adjacent the front edge (10) of the article, wherein the front waistband is attached directly or indirectly to the topsheet and at least partially vertically overlaps with the front adhesive zone.

12. The absorbent article according to any of the preceding claims, wherein the backsheet-to-core adhesive pattern comprises a series of longitudinally-extending adhesive lines, preferably wherein each adhesive line has spiral pattern.

13. The absorbent article according to any of the preceding claims, further comprising an urine indicator composition (200) disposed on the absorbent core-facing side of the backsheet in the second non-adhesive zone (114).

14. The absorbent article according to any of the preceding claims, wherein the length (d1) of the first non-adhesive zone (110) as measured on the longitudinal centerline 80 is in the range of from 1 cm to 20 cm, in particular in the range of from 2 cm to 12 cm.

15. An industrial process for making absorbent articles according to any of the preceding claims on a converting line, wherein the process comprises the step of:
applying the backsheet-to-core adhesive on a backsheet material, wherein the adhesive is applied by a plurality of adhesive nozzles (222a, 222b) aligned in cross-machine direction, wherein the adhesive nozzles are controlled to intermittently apply the adhesive according to the backsheet-to-core adhesive pattern according to any of the preceding claims.
